# DEMANDE DE BREVET EUROPEEN

(11) **EP 2 210 636 A1**
(43) Date de publication de la demande: **28.07.2010**
(21) Numéro de dépôt: 10151713.4
(22) Date de dépôt: 26.01.2010
(51) Int. Cl.: A61M 5/40, A61M 5/14

(54) **Dispositif compte-gouttes pour ligne d'administration d'un liquide à un patient**

(30) Priorité: 26.01.2009 FR 0950442
(71) Demandeur: Doran International, 69780 Toussieu (FR)
(72) Inventeur: Buisson, Philippe, 69780 TOUSSIEU (FR)
(74) Mandataire: Maureau, Philippe

(57) **Abrégé**

Ce dispositif compte-gouttes comporte un corps souple (6) délimitant une chambre compte-gouttes (7) destinée à contenir un liquide, un orifice de passage de liquide (9), et un obturateur (11) disposé dans la chambre compte-gouttes et mobile entre une position d'ouverture de l'orifice de passage permettant une administration de liquide au patient et une position d'obturation de l'orifice de passage empêchant une administration de liquide au patient. Le dispositif compte-gouttes (5) comporte un support souple (8) monté dans la chambre compte-gouttes (7) et délimitant l'orifice de passage de liquide (9), et l'obturateur est une membrane flottante souple (11) dont une portion (12) est solidaire du support (8).

## Description

La présente invention concerne un dispositif compte-gouttes pour ligne d'administration d'un liquide à un patient, et un perfuseur comportant un tel dispositif.

Un tel dispositif compte-gouttes comporte de façon connue un corps délimitant une chambre compte-gouttes destinée à contenir un liquide, un orifice de passage de liquide, et un obturateur flottant disposé dans la chambre compte-gouttes et mobile entre une position d'ouverture de l'orifice de passage permettant une administration de liquide au patient et une position d'obturation de l'orifice de passage empêchant une administration de liquide au patient.

Un inconvénient de ce type dispositif compte-gouttes réside dans le fait que l'obturateur flottant est susceptible de se « coller » contre la paroi intérieure de la chambre compte-gouttes lorsqu'il se trouve dans sa position d'ouverture de telle sorte qu'il ne peut se déplacer dans sa position d'obturation ou revenir correctement dans cette position lorsque le niveau de liquide dans la chambre compte-gouttes atteint le fond de cette dernière.

II en résulte un désamorçage du dispositif compte-gouttes et une introduction d'air dans la tubulure reliant le dispositif compte-gouttes à un cathéter relié à un patient, ce qui nécessitera une étape supplémentaire de purge de la tubulure afin de réamorcer le dispositif compte-gouttes.

La présente invention vis à remédier à ces inconvénients.

Le problème technique à la base de l'invention consiste donc à fournir un dispositif compte-gouttes pour ligne d'administration d'un liquide à un patient qui soit de structure simple et économique, tout en permettant d'éviter un désamorçage du dispositif durant son utilisation.

A cet effet, l'invention concerne un dispositif compte-gouttes pour ligne d'administration d'un liquide à un patient, le dispositif compte-gouttes comportant un corps délimitant une chambre compte-gouttes destinée à contenir un liquide, un orifice de passage de liquide, et un obturateur disposé dans la chambre compte-gouttes et mobile entre une position d'ouverture de l'orifice de passage permettant une administration de liquide au patient et une position d'obturation de l'orifice de passage empêchant une administration de liquide au patient, **caractérisé en ce que** le dispositif compte-gouttes comporte un support souple monté dans la chambre compte-gouttes et délimitant l'orifice de passage de liquide, en ce que l'obturateur est une membrane flottante souple dont une portion est solidaire du support, et en ce que le corps délimitant la chambre compte-gouttes est souple.

Le fait que l'obturateur soit une membrane flottante dont une portion est solidaire d'un support monté dans la chambre compte-gouttes et délimitant l'orifice de passage de liquide permet d'éviter que l'obturateur se « colle » contre la paroi de la chambre compte-gouttes lorsqu'il se trouve dans sa position d'ouverture, et donc d'assurer un bon positionnement de l'obturateur par rapport à l'orifice de passage de liquide lorsque l'obturateur atteint sa position d'obturation.

Par conséquent, le dispositif selon l'invention permet d'éviter un désamorçage de la chambre compte-gouttes durant son utilisation.

En outre, le fait que le support et le corps du dispositif soient souples permet de faciliter le déplacement de la membrane flottante de sa position d'obturation à sa position d'ouverture. En effet, lorsqu'un utilisateur exerce une pression sur le corps du dispositif au niveau du support, le corps du dispositif et le support se déforment de manière à décoller la membrane du support et à déplacer cette dernière dans sa position d'ouverture afin de permettre le passage de liquide à travers l'orifice de passage de liquide.

De préférence, la membrane flottante est agencée pour être déplacée par flottaison dans sa position d'ouverture lorsque le niveau de liquide dans la chambre compte-gouttes est situé au dessus du support et pour être déplacée par flottaison dans sa position d'obturation lorsque le niveau de liquide dans la chambre compte-gouttes est situé sensiblement au niveau du support. Ces dispositions permettent d'éviter que le niveau de liquide dans la chambre compte-gouttes descende en dessous d'un niveau prédéterminé de liquide, donc d'éviter encore un désamorçage du dispositif compte-gouttes.

Selon un mode de réalisation, la portion de la membrane solidaire du support est située sensiblement sur la périphérie de la membrane. De préférence, la portion de la membrane solidaire du support s'étend sur moins de la moitié de la périphérie de la membrane. Avantageusement, la membrane flottante est fixée directement sur le support, et la portion de la membrane flottante fixée sur le support est de préférence sensiblement ponctuelle.

Avantageusement, le support est fixé de manière étanche à la paroi intérieure ou aux parois intérieures du corps délimitant la chambre compte-gouttes, de préférence à distance des portions d'extrémité du corps.

Selon un mode de réalisation, le support comporte, sur sa face tournée vers la membrane flottante, une nervure s'étendant autour de l'orifice de passage de liquide et sur laquelle est destinée à reposer la membrane flottante lorsqu'elle est dans sa position d'obturation. Ces dispositions permettre d'améliorer l'étanchéité de la membrane flottante au niveau de l'orifice de passage de liquide lorsque cette dernière est dans sa position d'obturation.

Avantageusement, l'orifice de passage de liquide est délimité par une surface tronconique convergeant à l'opposé de la membrane flottante.

La présente invention concerne également un perfuseur pour ligne d'administration d'un liquide à un patient, **caractérisé en ce qu**'il comporte un dispositif compte-gouttes selon l'invention.

De toute façon l'invention sera bien comprise à l'aide de la description qui suit en référence au dessin schématique annexé représentant, à titre d'exemples non limitatifs, deux formes d'exécution de ce dispositif compte-gouttes.
Figure 1 est une vue de côté d'un perfuseur selon l'invention.
Figure 2 est une vue partielle en coupe d'un dispositif compte-gouttes selon un premier mode de réalisation de l'invention montrant la membrane flottante dans sa position d'ouverture.
Figure 3 est une vue partielle en coupe du dispositif compte-gouttes de figure 2 montrant la membrane flottante dans sa position d'obturation.
Figure 4 est une vue de dessous du support destiné à supporter la membrane flottante.
Figure 5 est une vue de dessus de la membrane flottante.
Figure 6 est une vue partielle en coupe d'un dispositif compte-gouttes selon un second mode de réalisation de l'invention montrant la membrane flottante dans sa position d'ouverture.
Figure 7 est une vue partielle en coupe du dispositif compte-gouttes de figure 4 montrant la membrane flottante dans sa position d'obturation.

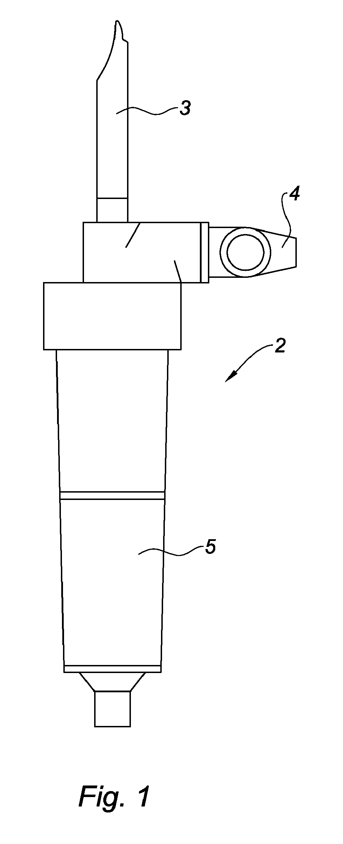

La figure 1 représente un perfuseur 2 pour ligne d'administration d'un liquide à un patient. Le perfuseur 2 comporte un perforateur 3 destiné à être raccordé à une poche de perfusion (non représentée sur les figures), une prise d'air 4 et un dispositif compte-gouttes 5 destiné à être raccordé à un prolongateur (non représenté sur les figures) équipé de moyens de connexion à un cathéter.

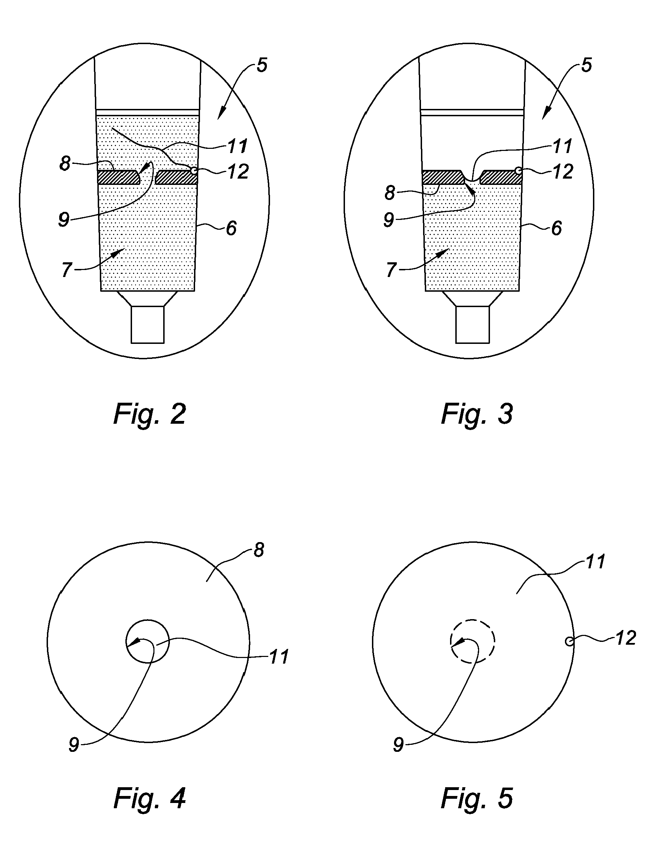

Comme montré plus particulièrement sur les figures 2 et 3, le dispositif compte-gouttes 5 comporte un corps souple 6 sensiblement transparent. Le corps souple 6 délimite une chambre compte-gouttes 7 destinée à contenir un liquide. Le corps souple 6 comporte une première portion d'extrémité tournée vers le perforateur 3 et une seconde portion d'extrémité opposée à la première portion d'extrémité, la seconde portion d'extrémité comportant des moyens de raccordement au prolongateur.

Le dispositif compte-gouttes 5 comporte en outre un support souple 8 fixé de manière étanche sur la paroi intérieure de la chambre compte-gouttes 7, à distance des portions d'extrémité du corps 6. Comme montré notamment sur la figure 4, le support 8 présente une forme circulaire et comporte un orifice de passage de liquide 9 ménagé en son centre. L'orifice de passage de liquide 9 est délimité par une surface sensiblement tronconique.

Le dispositif compte-gouttes 5 comporte également une membrane flottante souple 11 disposée dans la chambre compte-gouttes 7. La membrane flottante 11 présente une forme sensiblement circulaire. Une portion 12 de la membrane flottante 11, située sur la périphérie de cette dernière, est fixée sur le support 8. Cette portion 12 de la membrane flottante 11 est sensiblement ponctuelle, et peut être fixée sur le support 8 par exemple par collage.

La membrane flottante 11 est mobile entre une position d'ouverture de l'orifice de passage 9 (montrée sur la figure 2) permettant une administration de liquide au patient et une position d'obturation de l'orifice de passage 9 (montrée sur la figure 3) empêchant une administration de liquide au patient.

La membrane flottante 11 est agencée pour être déplacée par flottaison dans sa position d'ouverture lorsque le niveau de liquide dans la chambre compte-gouttes 8 est situé au dessus du support 8 et pour être déplacée par flottaison dans sa position d'obturation lorsque le niveau de liquide dans la chambre compte-gouttes est situé sensiblement au niveau du support 8.

II doit être noté que la surface tronconique délimitant l'orifice de passage de liquide 9 converge à l'opposé de la membrane flottante 11.

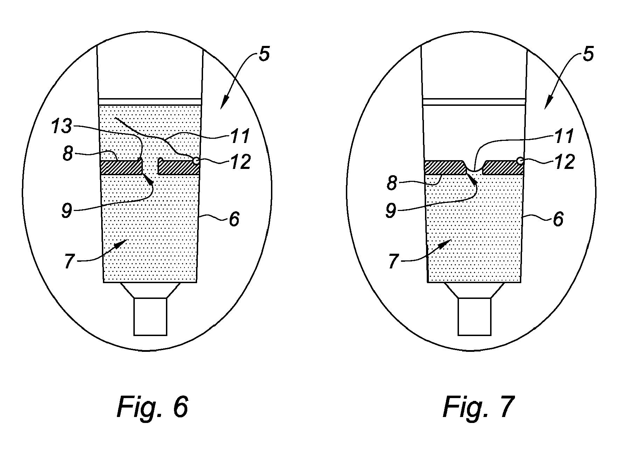

Les figures 6 et 7 représentent un dispositif compte-gouttes 5 selon un second mode de réalisation de l'invention qui diffère du dispositif représenté sur les figures 2 et 3 essentiellement en ce que le support souple 8 comporte, sur sa face tournée vers la membrane flottante 11, une nervure annulaire 13 s'étendant autour de l'orifice de passage de liquide 9 et sur laquelle est destinée à reposer la membrane flottante 11 lorsqu'elle est dans sa position d'obturation.

Comme il va de soi, l'invention ne se limite pas aux seules formes d'exécution de ce dispositif compte-gouttes, décrites ci-dessus à titre d'exemples, elle en embrasse au contraire toutes les variantes de réalisation.

## Revendications

1. Dispositif compte-gouttes (5) pour ligne d'administration d'un liquide à un patient, le dispositif compte-gouttes comportant un corps (6) délimitant une chambre compte-gouttes (7) destinée à contenir un liquide, un orifice de passage de liquide (9), et un obturateur (11) disposé dans la chambre compte-gouttes et mobile entre une position d'ouverture de l'orifice de passage permettant une administration de liquide au patient et une position d'obturation de l'orifice de passage empêchant une administration de liquide au patient, **caractérisé en ce que** le dispositif compte-gouttes (5) comporte un support souple (8) monté dans la chambre compte-gouttes (7) et délimitant l'orifice de passage de liquide (9), **en ce que** l'obturateur est une membrane flottante souple (11) dont une portion (12) est solidaire du support (8), et **en ce que** le corps (6) délimitant la chambre compte-gouttes est souple.

2. Dispositif selon la revendication 1, **caractérisé en ce que** la membrane flottante (11) est agencée pour être déplacée par flottaison dans sa position d'ouverture lorsque le niveau de liquide dans la chambre compte-gouttes (7) est situé au dessus du support (8) et pour être déplacée par flottaison dans sa position d'obturation lorsque le niveau de liquide dans la chambre compte-gouttes (7) est situé sensiblement au niveau du support (8).

3. Dispositif selon la revendication 1 ou 2, **caractérisé en ce que** la portion (12) de la membrane solidaire du support (8) est située sensiblement sur la périphérie de la membrane.

4. Dispositif selon la revendication 3, **caractérisé en ce que** la portion (12) de la membrane solidaire du support (8) s'étend sur moins de la moitié de la périphérie de la membrane.

5. Dispositif selon l'une des revendications 1 à 4, **caractérisé en ce que** le support (8) est fixé de manière étanche à la paroi intérieure ou aux parois intérieures du corps délimitant de la chambre compte-gouttes (7), de préférence à distance des portions d'extrémité du corps.

6. Dispositif selon l'une des revendications 1 à 5, **caractérisé en ce que** le support (8) comporte, sur sa face tournée vers la membrane flottante, une nervure (13) s'étendant autour de l'orifice de passage de liquide (9) et sur laquelle est destinée à reposer la membrane flottante (11) lorsqu'elle est dans sa position d'obturation.

7. Dispositif selon l'une des revendications 1 à 6, **caractérisé en ce que** l'orifice de passage de liquide (9) est délimité par une surface tronconique convergeant à l'opposé de la membrane flottante (11).

8. Perfuseur (2) pour ligne d'administration d'un liquide à un patient, **caractérisé en ce qu'**il comporte un dispositif compte-gouttes (5) selon l'une des revendications 1 à 7.
